(19) Europäisches Patentamt
European Patent Office
Office européen des brevets

(11) EP 2 095 809 A1

(12) DEMANDE DE BREVET EUROPEEN

(43) Date de publication:
**02.09.2009 Bulletin 2009/36**

(21) Numéro de dépôt: **09153980.9**

(22) Date de dépôt: **27.02.2009**

(51) Int Cl.:
***A61K 8/49*** (2006.01) ***A61K 8/58*** (2006.01)
***A61Q 5/06*** (2006.01) ***A61K 8/41*** (2006.01)
***A61K 8/40*** (2006.01)

(84) Etats contractants désignés:
**AT BE BG CH CY CZ DE DK EE ES FI FR GB GR HR HU IE IS IT LI LT LU LV MC MK MT NL NO PL PT RO SE SI SK TR**
Etats d'extension désignés:
**AL BA RS**

(30) Priorité: **28.02.2008 FR 0851300**

(71) Demandeur: **L'Oréal**
**75008 Paris (FR)**

(72) Inventeur: **De Boni, Maxime**
**Tokyo 162-0842 (JP)**

(74) Mandataire: **Wattremez, Catherine**
**L'OREAL**
**River Plaza - DIPI**
**25-29 Quai Aulagnier**
**92665 Asnières-sur-Seine (FR)**

# (54) Composition comprenant un colorant hydrophobe et un carbonate d'alkylène ou une lactone et coloration de fibres kératiniques

(57) La présente invention a pour objet des compositions utiles à la coloration de fibres kératiniques humaines, comprenant dans un milieu cosmétiquement acceptable, au moins un colorant à caractère hydrophobe et au moins un composé choisi parmi ceux de formule (I) suivante :

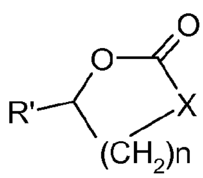

dans laquelle X représente O, $CH_2$ ; R' un hydrogène, un alkyle en $C_1$-$C_8$, un hydroxyalkyle en $C_1$-$C_4$ ; n vaut 1, 2 ou 3. Le composé de formule (I) peut être en particulier un carbonate d'alkylène ou une lactone ; le pH de la composition étant compris entre 2 et 6.

Elle a également pour objet un procédé de coloration de fibres kératiniques mettant en jeu une telle composition.

EP 2 095 809 A1

## Description

La présente invention a pour objet des compositions utiles à la coloration de fibres kératiniques humaines, comprenant dans un milieu cosmétiquement acceptable, au moins un colorant hydrophobe et au moins un composé choisi parmi les carbonates d'alkylène et/ou les lactones.

Elle a également pour objet un procédé de coloration de fibres kératiniques humaines mettant en jeu une telle composition.

On connaît deux grands modes de coloration des fibres kératiniques humaines, et en particulier les cheveux.

Le premier, appelé coloration d'oxydation ou permanente, consiste à mettre en oeuvre un ou plusieurs précurseurs de colorant d'oxydation, plus particulièrement une ou plusieurs bases d'oxydation éventuellement associées à un ou plusieurs coupleurs.

Habituellement, des bases d'oxydation sont choisies parmi les ortho- ou paraphénylènediamines, les ortho- ou para-aminophénols ainsi que des composés hétérocycliques. Ces bases d'oxydation sont des composés incolores ou faiblement colorés qui, associés à des produits oxydants, permettent d'accéder, par un processus de condensation oxydative, à des espèces colorées qui restent piégées à l'intérieur de la fibre.

Bien souvent, on fait varier les nuances obtenues avec ces bases d'oxydation en les associant à un ou plusieurs coupleurs, ces derniers étant choisis notamment parmi les méta-diamines aromatiques, les méta-aminophénols, les méta-diphénols et certains composés hétérocycliques, tels que des composés indoliques.

La variété des molécules mises en jeu au niveau des bases d'oxydation et des coupleurs permet l'obtention d'une riche palette de couleurs.

Le deuxième mode de coloration, appelé coloration directe ou semi-permanente, comprend l'application de colorants directs qui sont des molécules colorées et colorantes, ayant une affinité pour les fibres. Etant donnée la nature des molécules employées, celles-ci restent plutôt en surface de la fibre et pénètrent relativement peu à l'intérieur de la fibre, comparées aux petites molécules de précurseurs de colorants d'oxydation.

Les colorants directs généralement employés sont choisis parmi les colorants directs nitrés benzéniques, anthraquinoniques, nitropyridiniques, azoïques, méthiniques, azométhiniques, xanthéniques, acridiniques, aziniques ou triarylméthaniques. Les espèces chimiques mises en oeuvre peuvent être non ioniques, anioniques (colorants acides) ou cationiques (colorants basiques).

La plupart des colorants directs mis en oeuvre ont une solubilité en milieu aqueux suffisante et il existe maintenant de nombreux supports de coloration adaptés pour les recevoir.

La situation est différente dans le cas de colorants à caractère hydrophobe.

En effet, pour colorer efficacement les fibres kératiniques, certains de ces colorants hydrophobes doivent être dans la majeure partie des cas, employés en présence de solvants dont le rôle est de les vectoriser dans la fibre. Parmi les solvants cosmétiques connus pour cet effet, on utilise fréquemment les solvants aromatiques. On peut citer plus particulièrement l'alcool benzylique, le benzyloxyéthanol ou encore le phénoxyéthanol.

Néanmoins, la présence d'alcools aromatiques dans ces formulations amplifie le problème de tachage du cuir chevelu et de la peau rencontré en coloration.

En outre, et cela représente un autre inconvénient à l'utilisation de tels colorants directs, la présence des solvants aromatiques, très peu solubles en milieu aqueux, nécessite l'emploi de quantités importantes de co-solvants (habituellement l'éthanol) pour les rendre compatibles avec les formulations tinctoriales classiques.

L'un des objectifs de la présente invention est donc de proposer des compositions tinctoriales comprenant au moins un colorant direct à caractère hydrophobe et qui présentent des propriétés tinctoriales satisfaisantes, notamment en ce qu'elles permettent d'obtenir des colorations puissantes et homogènes entre la pointe et la racine d'une même fibre et d'une fibre à l'autre.

Ces objectifs et d'autres sont atteints par la présente invention qui a pour objet des compositions tinctoriales comprenant dans un milieu cosmétiquement acceptable, au moins un ou plusieurs colorants directs hydrophobes de logP supérieur ou égal à 2 et un ou plusieurs composés choisis parmi ceux de formule (I) suivante :

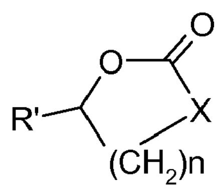

formule dans laquelle X représente un atome d'oxygène ou un groupement $CH_2$, R' un atome d'hydrogène, un radical alkyle linéaire ou ramifié en $C_1$-$C_8$, un radical hydroxyalkyle linéaire ou ramifié en $C_1$-$C_4$; n vaut 1, 2 ou 3 ; le pH de la

composition étant compris entre 2 et 6.

Elle a également pour objet un procédé de coloration de fibres kératiniques humaines, et en particulier les cheveux, consistant à appliquer sur lesdites fibres, la composition selon l'invention.

D'autres caractéristiques et avantages de l'invention apparaîtront plus clairement à la lecture de la description et des exemples.

Il est à noter que dans ce qui va suivre, et à moins d'une autre indication, les bornes d'un domaine de valeurs sont comprises dans ce domaine.

Comme indiqué précédemment, la composition tinctoriale selon l'invention comprend au moins un colorant direct à caractère hydrophobe de logP supérieur ou égal à 2.

La valeur du logP représente de façon classique le coefficient de partage du colorant entre l'octanol et l'eau. Le logP peut être calculé selon la méthode décrite dans l'article de Meylan et Howard « Atom / Fragment contribution method for estimating octano/-water partition coefficient », J. Pharm. Sci. 84 :83-92, 1995. Cette valeur peut aussi être calculée à partir de nombreux logiciels disponibles sur le marché qui détermine le logP en fonction de la structure d'une molécule. A titre d'exemple, on peut citer le logiciel Epiwin de l'agence de l'environnement des Etats-Unis.

En particulier, les colorants convenant à la mise en oeuvre de l'invention sont choisis parmi les composés suivants, seuls ou en mélange :

| Colorant | Structure chimique | logP |
|---|---|---|
| Disperse Red 17 | 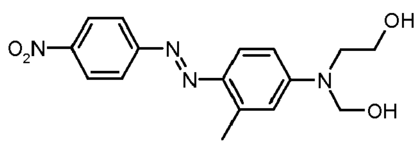 | 3.69 |
| Disperse Violet 1 | 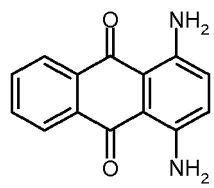 | 3.0 |
| HC Yellow 7 | 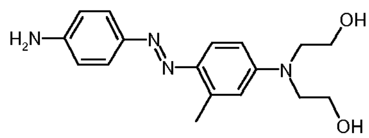 | 2.38 |
| Disperse Blue 377 | 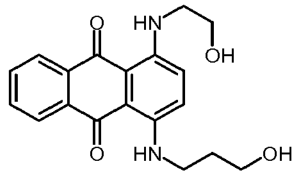 | 3.21 |
| Disperse Red 13 | 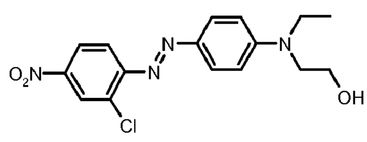 | 5.22 |
| Disperse Green 9 | 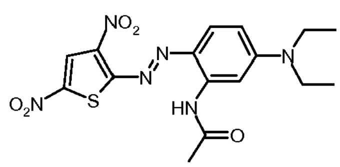 | 4.23 |

(suite)

| Colorant | Structure chimique | logP |
|---|---|---|
| Solvent Black 3 | 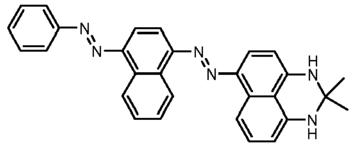 | 7.50 |
| Disperse Blue 148 | 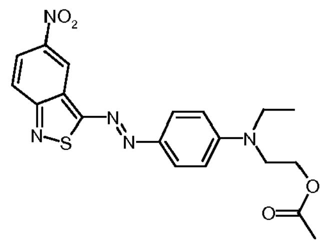 | 4.81 |
| Disperse Violet 63 | 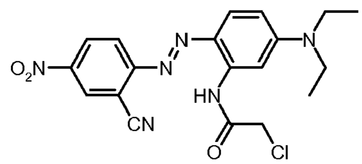 | 5.30 |
| Disperse Blue 60 | 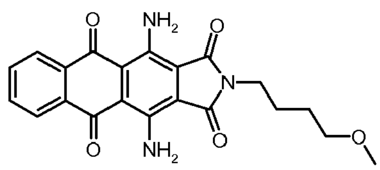 | 3.38 |
| Disperse Blue 14 | 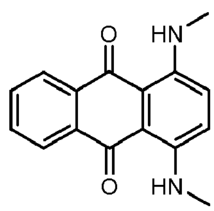 | 4.25 |
| Solvent Orange 15 | 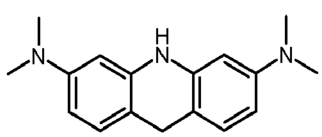 | 3.90 |
| Solvent Orange 7 | 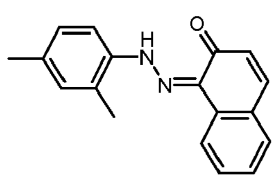 | 4.40 |
| Solvent Blue 14 | 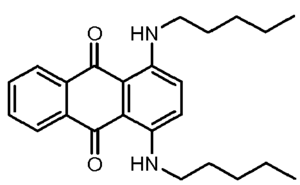 | 8.18 |

(suite)

| Colorant | Structure chimique | logP |
|---|---|---|
| Disperse Yellow 82 | 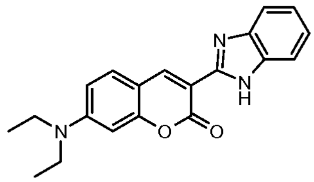 | 3.68 |

Selon un mode de réalisation particulier de l'invention, la teneur en colorant(s) direct(s) hydrophobe(s) varie de 0,01 à 10 % en poids, plus particulièrement de 0,5 à 10% en poids, par rapport au poids de la composition, de préférence de 0,01 à 5% en poids par rapport au poids de la composition.
La composition tinctoriale selon l'invention comprend également au moins un composé choisis parmi ceux de formule (I) précitée.
Selon une première variante, le composé de formule (I) est tel que X représente un atome d'oxygène et n vaut 1.
Ainsi, le composé est un carbonate d'alkylène et correspond à un composé de formule (Ia) suivante :

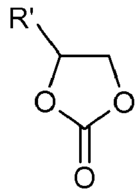

dans laquelle R' représente un atome d'hydrogène, un radical alkyle linéaire ou ramifié en $C_1$-$C_8$, un radical hydroxyalkyle linéaire ou ramifié en $C_1$-$C_4$.
De préférence, le radical R' représente un atome d'hydrogène, un radical alkyle linéaire ou ramifié en $C_1$-$C_4$, un radical hydroxyalkyle linéaire ou ramifié en $C_1$-$C_2$.
A titre d'exemples particulièrement avantageux de carbonate d'alkylène, on peut citer les composés pour lesquels le radical R' représente un atome d'hydrogène (correspondant au carbonate d'éthylène), un groupement méthylène (correspondant au carbonate de propylène), éthylène (correspondant au carbonate de butylène), hydroxyméthylène (R' = $-CH_2OH$ ; correspondant au carbonate de glycérine).
De préférence, le carbonate d'alkylène mis en oeuvre est le carbonate de propylène.
Selon une deuxième variante de l'invention, le composé de formule (I) est tel que X représente un groupe $CH_2$. Dans le cadre de cette variante, n vaut1, 2 ou 3.
En particulier, le composé correspond à une lactone de formule (Ib) suivante :

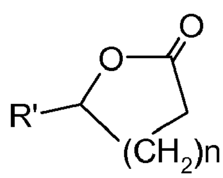

dans laquelle R' représente un atome d'hydrogène, un radical alkyle linéaire ou ramifié en $C_1$-$C_8$, un radical hydroxyalkyle linéaire ou ramifié en $C_1$-$C_4$, n vaut 1, 2 ou 3.
De préférence, le radical R' représente un atome d'hydrogène, un radical alkyle linéaire ou ramifié en $C_1$-$C_6$, un radical hydroxyalkyle linéaire ou ramifié en $C_1$-$C_2$.
Selon une premier mode de réalisation, la ou les lactones sont telles que le radical R' représente un groupement méthylène (correspondant à la γ-valérolactone), éthylène (correspondant à la γ-caprolactone), n-propylène (correspondant à la γ-heptanolactone), n-butylène (correspondant à la γ-octanolactone), hydroxyméthylène (R' = $-CH_2OH$ ; correspondant à la dihydro-5-hydroxyméthyl)-2(3H)-furanone), avec n valant 1.
Selon une deuxième variante, la ou les lactones sont telles que le radical R' représente un atome d'hydrogène (correspondant à la δ-valérolactone), un groupement n-butylène (correspondant à la δ-nonalactone), avec n valant 2.

Selon une troisième variante, la ou les lactones sont telles que le radical R' représente un atome d'hydrogène avec n valant 3, correspondant à la ε-caprolactone

De préférence, la lactone de formule (Ib) est la γ-caprolactone.

Selon un mode de réalisation particulièrement avantageux de l'invention, la composition tinctoriale comprend, un ou plusieurs carbonate d'alkylène de formule (la) et de préférence, le carbonate d'éthylène.

Conformément à une variante particulière de l'invention, la teneur en composé(s) de formule (I) représente de 0,5 à 50% en poids par rapport au poids de la composition, et de préférence de 1 à 30% en poids, par rapport au poids de la composition.

La composition selon l'invention peut également comprendre un ou plusieurs colorants supplémentaires. En particulier, ce ou ces colorants supplémentaires peuvent être choisis parmi les colorants naturels, les colorants directs différents des colorants à caractère hydrophobe, les précurseurs de colorant d'oxydation, ou leurs combinaisons.

Par colorants naturels, on entend tout colorant ou précurseur de colorant ayant une occurrence naturelle et produit soit par extraction (et éventuellement purification) depuis une matrice végétale, soit par synthèse chimique.

Les colorants naturels convenables en particulier à la mise en oeuvre de l'invention sont choisis parmi la lawsone, la juglone, l'alizarine, la purpurine, l'acide carminique, l'acide kermésique, la purpurogalline, la protocatéchaldéhyde, l'indigo, l'isatine, la curcumine, la spinusoline, les chlorophylles, les chlorophyllines, les orcéines, l'hématéine, l'hématoxyline, la braziline, la braziléine, les colorants du carthame (comme par exemple la carthamine), les flavonoïdes (avec par exemple la morine, l'apigénidine, le santal), les anthocyanes (du type de l'apigéninidine), les caroténoïdes, les tanins, le sorgho et le carmin de cochenille.

On peut également utiliser les extraits ou décoctions contenant ces colorants naturels et notamment les extraits à base de henné.

De préférence, le colorant naturel est choisi parmi la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine, la chlorophylline, le sorgho, les orcéines et le carmin de cochenille.

Les colorant naturels, lorsqu'ils sont présents, représentent de 0,01 à 10% en poids par rapport au poids, de préférence de 0,1 à 5% en poids par rapport au poids de la composition.

La composition peut également comprendre un ou plusieurs colorants directs additionnels différents des colorants à caractère hydrophobe décrits plus haut, et choisi parmi des espèces ioniques ou non ioniques, de préférence cationiques ou non ioniques.

A titre d'exemples de colorants directs additionnel convenables, on peut citer les colorants directs azoïques ; méthiniques ; carbonyles ; aziniques ; nitrés (hétéro)aryle ; tri-(hétéro)aryle méthanes ; les porphyrines et les phtalocyanines, seuls ou en mélanges.

Plus particulièrement, les colorants azoïques comprennent une fonction -N=N- dont les deux atomes d'azote ne sont pas simultanément engagés dans un cycle. Il n'est toutefois pas exclu que l'un des deux atomes d'azote de l'enchaînement -N=N- soit engagé dans un cycle.

Les colorants de la famille des méthines sont plus particulièrement des composés comprenant au moins un enchaînement choisi parmi >C=C< et -N=C< dont les deux atomes ne sont pas simultanément engagés dans un cycle. Il est toutefois précisé que l'un des atomes d'azote ou de carbone des enchaînements peut être engagé dans un cycle. Plus particulièrement, les colorants de cette famille sont issus de composés de type méthine, azométhine, mono- et diarylméthane, indoamines (ou diphénylamines), indophénols, indoanilines, carbocyanines, azacabocyanines et leurs isomères, diazacarbocyanines et leurs isomères, tétraazacarbocyanines, hémicyanines

Concernant les colorants de la famille des carbonyles, on peut citer par exemple les colorants choisis parmi les acridone, benzoquinone, anthraquinone, naphtoquinone, benzanthrone, anthranthrone, pyranthrone, pyrazolanthrone, pyrimidinoanthrone, flavanthrone, idanthrone, flavone, (iso)violanthrone, isoindolinone, benzimidazolone, isoquinolinone, anthrapyridone, pyrazoloquinazolone, périnone, quinacridone, quinophthalone, indigoïde, thioindigo, naphtalimide, anthrapyrimidine, dicétopyrrolopyrrole, coumarine.

Concernant les colorants de la famille des azines cycliques, on peut citer notamment les azine, xanthène, thioxanthène, fluorindine, acridine, (di)oxazine, (di)thiazine, pyronine.

Les colorants nitrés (hétéro)aromatiques sont plus particulièrement des colorants directs nitrés benzéniques ou nitrés pyridiniques.

Concernant les colorants de type porphyrines ou phtalocyanines, on peut mettre en oeuvre des composés cationiques ou non, comprenant éventuellement un ou plusieurs métaux ou ions métalliques, comme par exemple des métaux alcalins et alcalino-terreux, le zinc et le silicium.

A titre d'exemple de colorants directs de synthèse additionnel particulièrement convenables, on peut citer les colorants nitrés de la série benzénique ; les colorants directs azoïques ; azométhiniques; méthiniques; les azacarbocyanines comme les tétraazacarbocyanines (tétraazapentaméthines) ; les colorants directs quinoniques et en particulier anthraquinoniques, naphtoquinoniques ou benzoquinoniques; les colorants directs aziniques; xanthéniques ; triarylméthaniques ; indoaminiques; indigoïdes; phtalocyanines et porphyrines; seuls ou en mélanges. De manière encore plus préférée, ces colorants directs additionnels sont choisis parmi les colorants nitrés de la série benzénique ; les colorants directs azoïques ; azométhiniques; méthiniques et les tétraazacarbocyanines (tétraazapentaméthines) ; seuls ou en mélange.

Ces colorants peuvent être des colorants monochromophoriques (c'est-à-dire ne comprenant qu'un seul colorant) ou polychromophoriques, de préférence di- ou tri-chromophoriques; les chromophores pouvant être identiques ou non, de la même famille chimique ou non. A noter que qu'un colorant polychromophorique comprend plusieurs radicaux issus chacun d'une molécule absorbant dans le domaine visible entre 400 et 800 nm. De plus cette absorbance du colorant ne nécessite ni oxydation préalable de celui-ci, ni association avec d'autre(s) espèce(s) chimique(s).

Dans le cas de colorants polychromophoriques, les chromophores sont reliés entre eux au moyen d'au moins un bras de liaison qui peut être cationique ou non.

Parmi les colorants polychromophoriques, on peut citer plus particulièrement les colorants di- ou tri- chromophoriques azoïques et/ou azométhiniques (hydrazoniques), symétriques ou non, comprenant d'une part au moins un hétérocycle aromatique comprenant 5 ou 6 chaînons, éventuellement condensé, comprenant au moins un atome d'azote quaternisé engagé dans ledit hétérocycle et éventuellement au moins un autre hétéroatome (tel que l'azote, le soufre, l'oxygène), et d'autre part, au moins un groupement phényle ou naphtyle, éventuellement substitué, éventuellement porteur d'au moins un groupement OR avec R représentant un atome d'hydrogène, un radical alkyle éventuellement substitué en $C_1$-$C_6$, un noyau phényle éventuellement substitué, ou d'au moins un groupement $N(R')_2$ avec R' identiques ou non, représentant un atome d'hydrogène, un radical alkyle éventuellement substitué en $C_1$-$C_6$, un noyau phényle éventuellement substitué ; les radicaux R' pouvant former avec l'atome d'azote auquel ils sont liés, un hétérocycle saturé à 5 ou 6 chaînons, ou bien encore l'un et/ou les deux radicaux R' peuvent former chacun avec l'atome de carbone du cycle aromatique placé en ortho de l'atome d'azote, un hétérocycle saturé à 5 ou 6 chaînons.

A titre d'hétérocycle cationique aromatique, on peut citer de préférence, les cycles à 5 ou 6 chaînons comprenant 1 à 3 atomes d'azote, de préférence 1 ou 2 atomes d'azote, l'un étant quaternisé ; ledit hétérocycle étant par ailleurs éventuellement condensé à un noyau benzénique. Il est à noter de même que l'hétérocycle peut éventuellement comprendre un autre hétéroatome différent de l'azote, comme le soufre ou l'oxygène.

Si les hétérocycles ou groupements phényle ou napthyle sont substitués, ils le sont par exemple par un ou plusieurs radicaux alkyle en $C_1$-$C_8$ éventuellement substitués par un groupement hydroxy, alcoxy en $C_1$-$C_2$, hydroxyalcoxy en $C_2$-$C_4$, acétylamino, amino substitué par un ou deux radicaux alkyle en $C_1$-$C_4$, éventuellement porteurs d'un groupement hydroxyle ou les deux radicaux pouvant former avec l'atome d'azote auquel ils sont rattachés, un hétérocycle à 5 ou 6 chaînons, comprenant éventuellement un autre hétéroatome identique ou différent de l'azote ; un atome d'halogène ; un groupement hydroxyle; un radical alcoxy en $C_1$-$C_2$; un radical hydroxyalcoxy en $C_2$-$C_4$; un radical amino ; un radical amino substitué par un ou deux radicaux alkyle, identiques ou différents, en $C_1$-$C_4$ éventuellement porteurs d'un groupement hydroxyle.

De préférence, le bras de liaison est une chaîne alkyle en $C_1$-$C_{20}$, linéaire, ramifiée ou cyclique; éventuellement interrompue par au moins un hétéroatome (tel que l'azote, l'oxygène) et/ou par au moins un groupe en comprenant (CO, $SO_2$); éventuellement interrompue par au moins un groupement phényle ou naphtyle substitué ou non ; éventuellement interrompue par au moins un hétérocycle saturé, insaturé ou aromatique, condensé ou non avec un noyau phényle, ledit hétérocycle comprenant au moins un atome d'azote quaternisé engagé dans ledit cycle et éventuellement au moins un autre hétéroatome (tel que l'oxygène, l'azote ou le soufre) ; éventuellement interrompue par au moins un groupement ammonium quaternaire substitué par deux groupements alkyle en $C_1$-$C_{15}$ ; le bras de liaison ne comprenant pas de groupement nitro, nitroso ou peroxo.

Si les hétérocycles ou noyaux aromatiques sont substitués, ils le sont par exemple par un ou plusieurs radicaux alkyle en $C_1$-$C_8$ éventuellement substitués par un groupement hydroxy, alcoxy en $C_1$-$C_2$, hydroxyalcoxy en $C_2$-$C_4$, acétylamino, amino substitué par un ou deux radicaux alkyle, identiques ou différents, en $C_1$-$C_4$, éventuellement porteurs d'un groupement hydroxyle ou les deux radicaux pouvant former avec l'atome d'azote auquel ils sont rattachés, un hétérocycle à 5 ou 6 chaînons, comprenant éventuellement un autre hétéroatome identique ou différent de l'azote ; un atome d'halogène ; un groupement hydroxyle; un radical alcoxy en $C_1$-$C_2$; un radical hydroxyalcoxy en $C_2$-$C_4$; un radical amino ; un radical amino substitué par un ou deux radicaux alkyle, identiques ou différents, en $C_1$-$C_4$ éventuellement porteurs d'un groupement hydroxyle.

La liaison entre le bras de liaison et chaque chromophore se fait en général au moyen d'un hétéroatome substituant le noyau phényle ou napthyle ou au moyen de l'atome d'azote quaternisé de l'hétérocycle cationique.

Parmi les colorants directs monochromophoriques azoïques, azométhines, méthines ou tétraazapentaméthines utilisables selon l'invention on peut citer les colorants cationiques décrits dans les demandes de brevets WO 95/15144, WO 95/01772 et EP 714954 ; FR 2189006, FR 2285851, FR-2140205, EP 1378544, EP 1674073.

Ainsi, on peut tout notamment citer les colorants directs cationiques correspondants aux formules suivantes :

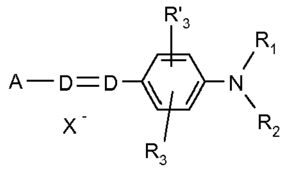

dans laquelle :

D représente un atome d'azote ou le groupement -CH,

$R_1$ et $R_2$, identiques ou différents, représentent un atome d'hydrogène ; un radical alkyle en $C_1$-$C_4$ pouvant être substitué par un radical -CN, -OH ou -$NH_2$ ou forment avec un atome de carbone du cycle benzénique un hétérocycle éventuellement oxygéné ou azoté, pouvant être substitué par un ou plusieurs radicaux alkyle en $C_1$-$C_4$ ; un radical 4'-aminophényle,

$R_3$ et $R'_3$, identiques ou différents, représentent un atome d'hydrogène ou d'halogène choisi parmi le chlore, le brome, l'iode et le fluor, un radical cyano, alkyl en $C_1$-$C_4$ , alcoxy en $C_1$-$C_4$ ou acétyloxy,

X - représente un anion de préférence choisi parmi le chlorure, le méthyl sulfate et l'acétate,

A représente un groupement choisi par les structures suivantes :

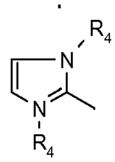 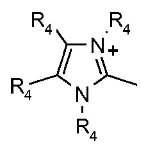 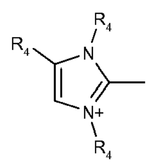 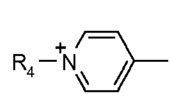 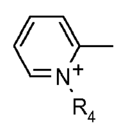

dans lesquelles $R_4$ représente un radical alkyle en $C_1$-$C_4$ pouvant être substitué par un radical hydroxyle;

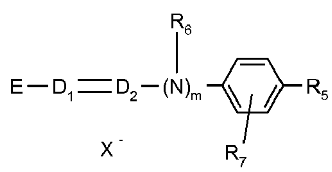

dans lesquelles :

$R_5$ représente un atome d'hydrogène, un radical alcoxy en $C_1$-$C_4$, un atome d'halogène tel que le brome, le chlore, l'iode ou le fluor,

$R_6$ représente un atome d'hydrogène, un radical alkyle en $C_1$-$C_4$ ou forme avec un atome de carbone du cycle benzénique un hétérocycle éventuellement oxygéné et/ou substitué par un ou plusieurs groupements alkyle en $C_1$-$C_4$,

$R_7$ représente un atome d'hydrogène ou d'halogène tel que le brome, le chlore, l'iode ou le fluor,

$D_1$ et $D_2$, identiques ou différents, représentent un atome d'azote ou le groupement -CH,

m = 0 ou 1,

$X^-$ représente un anion cosmétiquement acceptable et de préférence choisi parmi le chlorure, le méthyl sulfate et l'acétate,

E représente un groupement choisi par les structures suivantes :

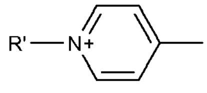 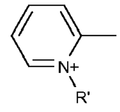 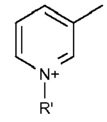

dans lesquelles R' représente un radical alkyle en $C_1$-$C_4$ ;

lorsque m = 0 et que $D_1$ représente un atome d'azote, alors E peut également désigner un groupement de structure suivante :

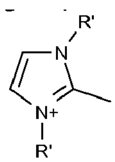

;

dans laquelle R' représente un radical alkyle en $C_1$-$C_4$.

Parmi les composés précités, on utilise tout particulièrement les composés suivants :

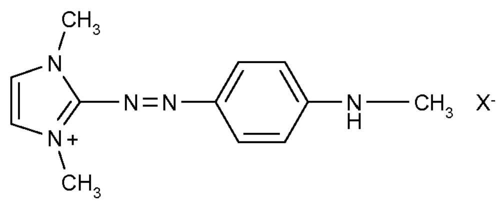

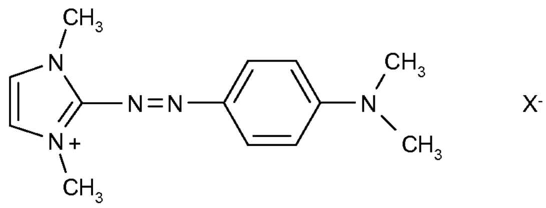

,

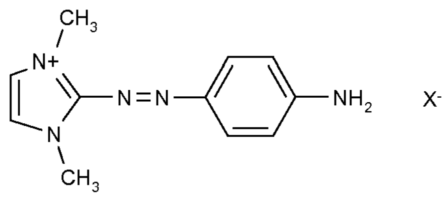

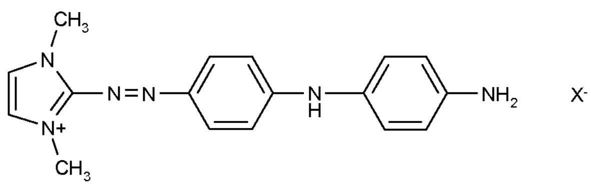

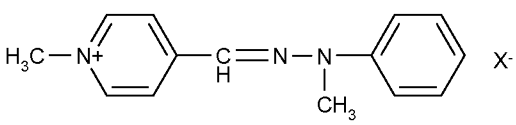

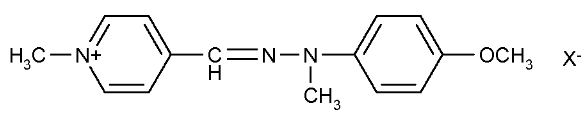

;

Parmi les colorants de type tétraazapentaméthiniques utilisables selon l'invention, on peut citer les composés suivants figurant dans le tableau ci-dessous :

| | |
|---|---|
| 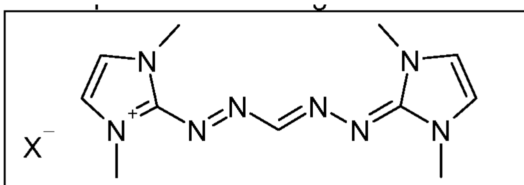 | 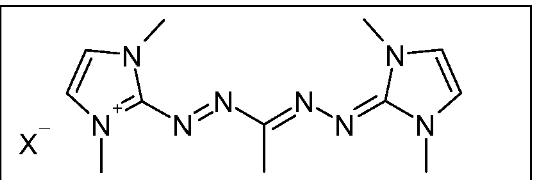 |
| 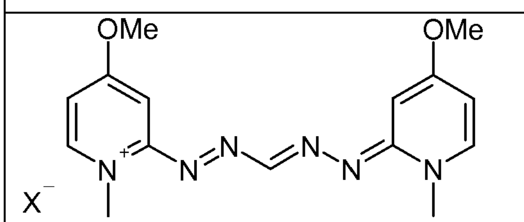 | 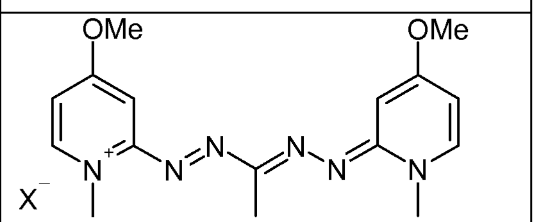 |
| 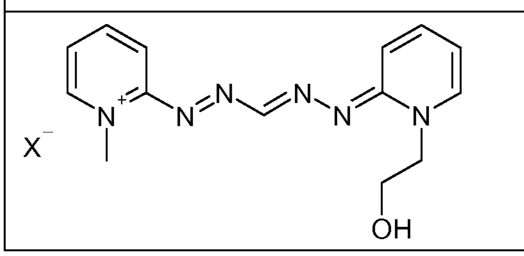 | 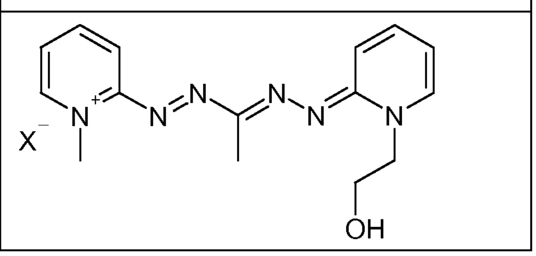 |

| | |
|---|---|
| 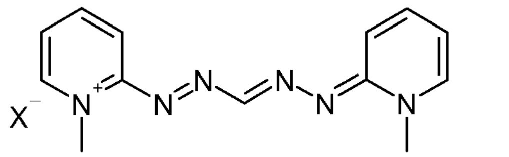 | 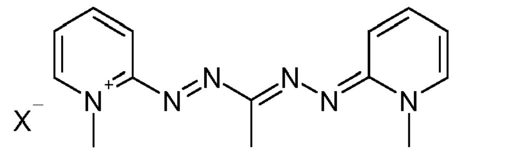 |
| 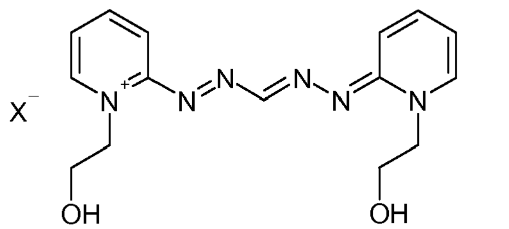 | 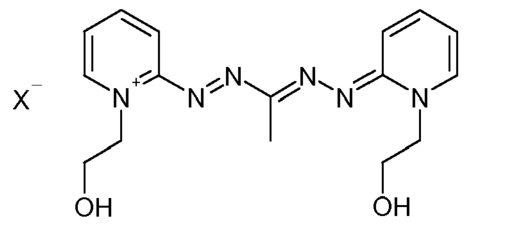 |

$X^-$ représente un anion de préférence choisi parmi le chlorure, l'iodure, le méthyl sulfate, l'éthyl sulfate, l'acétate.

Lorsqu'ils sont présents, le ou les colorants directs additionnels de synthèse représentent de 0,0001 à 10% en poids par rapport au poids de la composition, et de préférence de 0,005 à 5 % en poids par rapport à la même référence.

La composition tinctoriale peut également comprendre un ou plusieurs précurseurs de colorants d'oxydation, plus particulièrement une ou plusieurs bases d'oxydation éventuellement combinée(s) à un ou plusieurs coupleurs.

A titre d'exemple, les bases d'oxydation sont choisies parmi les paraphénylènediamines, les bis-phénylalkylènediamines, les para-aminophénols, les ortho-aminophénols, les bases hétérocycliques et leurs sels d'addition.

A titre d'exemple, les bases d'oxydation sont choisies parmi les paraphénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine,

et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a] pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4-5-diamino 1-(β-méthoxyéthyl)pyrazole.

A titre de dérivés pyrazoliques on peut également citer les diamino N,N-dihydropyrazolopyrazolones et notamment celles décrites dans la demande FR 2886136 telles que les composés suivants et leurs sels d'addition : 2,3-Diamino-6,7-dihydro-1H,5H-pyrazolo[1,2-a]pyrazol-1-one, 2-Amino-3-éthylamino-6,7-dihydro-1H,5H-pyrazolo[1,2-a] pyrazol-1-one, 2-Amino-3-isopropylamino-6,7-dihydro-1H,5H-pyrazolo[1,2-a]pyrazol-1-one, 2-Amino-3-(pyrrolidin-1-yl)-6,7-dihydro-1H,5H-pyrazolo[1,2-a]pyrazol-1-one, 4,5-diamino-1,2-diméthyl-1,2-dihydro-pyrazol-3-one, 4,5-diamino-1,2-diéthyl-1,2-dihydro-pyrazol-3-one, 4,5-diamino-1,2-di-(2-hydroxyéthyl)-1,2-dihydro-pyrazol-3-one, 2-amino-3-(2-hydroxyéthyl)amino-6,7-dihydro-1H,5H-pyrazolo[1,2-a]pyrazol-1-one, 2-amino-3-diméthylamino-6,7-dihydro-1H,5H-pyrazolo[1,2-a]pyrazol-1-one, 2,3-diamino-5,6,7,8-tétrahydro-1H,6H-pyridazino[1,2-a]pyrazol-1-one, 4-Amino-1,2-diethyl-5-(pyrrolidin-1-yl)-1,2-dihydro-pyrazol-3-one, 4-Amino-5-(3-dimethylamino-pyrrolidin-1-yl)-1,2-diethyl-1,2-dihydro-pyrazol-3-one, 2,3-diamino-6-hydroxy-6,7-dihydro-1H,5H-pyrazolo[1,2-a]pyrazol-1-one.

A titre de bases hétérocycliques on utilisera préférentiellement le 4,5-diamino 1-(β-hydroxyéthyl) pyrazole et/ou la 2,3-diamino-6,7-dihydro-1H,5H-pyrazolo[1,2,A]pyrazol-1-one et leurs sels d'addition.

La composition selon l'invention peut éventuellement comprendre un ou plusieurs coupleurs choisis avantageusement parmi ceux conventionnellement utilisés pour la teinture des fibres kératiniques.

Parmi ces coupleurs, on peut notamment citer les méta-phénylènediamines, les méta-aminophénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, leurs sels d'addition avec un acide, et leurs mélanges.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

La ou les bases d'oxydation, quand elles sont présentes dans la composition, représentent avantageusement de 0,0001 à 10% en poids par rapport au poids de la composition, et de préférence de 0,005 à 5 % en poids par rapport au poids de la composition.

La teneur en coupleur(s), s'il(s) est(sont) présent(s), représente avantageusement de 0,0001 à 10% en poids par rapport au poids de la composition, et de préférence de 0,005 à 5% en poids par rapport au poids de la composition.

La composition tinctoriale selon l'invention peut également comprendre un ou plusieurs agents de conditionnement.

A titre d'exemple, on peut citer les silicones linéaires, cyclique, ramifiées ou non ramifiées, volatiles ou non volatiles. Ces silicones peuvent se présenter sous forme d'huiles, de résines ou de gommes, elles peuvent en particulier être des polyorganosiloxanes insolubles dans le milieu cosmétiquement acceptable.

Les organopolysiloxanes sont définis plus en détail dans l'ouvrage de Walter NOLL "Chemistry and Technology of Silicones" (1968) Academic Press. Ils peuvent être volatils ou non volatils.

Lorsqu'elles sont volatiles, les silicones sont plus particulièrement choisies parmi celles possédant un point d'ébullition compris entre 60°C et 260°C.

A titre d'agent de conditionnement, on peut aussi utiliser les polymères cationiques tels que les polyquaterniums 22, 6, 10, 11, 35 et 37 et le chlorure d'hexadimethrine.

La concentration en agent(s) de conditionnement dans la ou les compositions utiles dans l'invention peut varier de 0,01 à 10% en poids par rapport au poids total de la composition, de préférence de 0,05 à 5% et plus préférentiellement encore de 0, 1 à 3%.

Les compositions selon l'invention peuvent contenir en outre un ou plusieurs agents épaississants organiques.

Les agents épaississants organiques peuvent être choisis parmi les amides d'acides gras (diéthanol- ou monoéthanol-amide de coprah, monoéthanolamide d'acide alkyl éther carboxylique oxyéthyléné), les épaississants polymériques tels que les épaississants cellulosiques (hydroxyéthycellulose, hydroxypropylcellulose, carboxyméthylcellulose), la gomme de guar et ses dérivés (hydroxypropylguar), les gommes d'origine microbienne (gomme de xanthane, gomme de scléroglucane), les homopolymères réticulés d'acide acrylique ou d'acide acrylamidopropanesulfonique et les polymères associatifs (polymères comprenant des zones hydrophiles, et des zones hydrophobes à chaîne grasse capables, dans un milieu aqueux, de s'associer réversiblement entre eux ou avec d'autres molécules.).

Selon un mode de réalisation particulier, l'épaississant est polymérique et choisi parmi les épaississants cellulosiques (hydroxyéthycellulose, hydroxypropylcellulose, carboxyméthylcellulose), la gomme de guar et ses dérivés (hydroxypropylguar), les gommes d'origine microbienne (gomme de xanthane, gomme de scléroglucane), les homopolymères réticulés d'acide acrylique ou d'acide acrylamidopropanesulfonique.

En ce qui concerne les agents épaississants associatifs, on peut mettre en oeuvre un ou plusieurs polymères de nature non ionique ou ionique, de préférence anionique ou cationique.

Leur structure chimique comprend plus particulièrement au moins une zone hydrophile et au moins une zone hydrophobe. Par groupement hydrophobe, on entend un radical ou polymère à chaîne hydrocarbonée, saturée ou non, linéaire ou ramifiée, comprenant au moins 8 atomes de carbone, de préférence de 10 à 30 atomes de carbone, en particulier de 12 à 30 atomes de carbone et plus préférentiellement de 18 à 30 atomes de carbone.

Préférentiellement, le groupement hydrocarboné provient d'un composé monofonctionnel.

A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Parmi les polymères amphiphiles anioniques comportant au moins une chaîne grasse (hydrophobe), on peut citer :

-(I) les polymères comportant au moins un motif hydrophile, et au moins un motif éther d'allyle à chaîne grasse, plus particulièrement ceux dont le motif hydrophile est constitué par un monomère anionique insaturé éthylénique, avantageusement par un acide carboxylique vinylique et tout particulièrement par un acide acrylique ou un acide méthacrylique ou les mélanges de ceux ci, et dont le motif éther d'allyle à chaîne grasse correspond au monomère de formule (A) suivante :

$$CH_2 = C\ R'\ CH_2\ O\ B_n\ R \qquad (A)$$

dans laquelle R' désigne H ou $CH_3$, B désigne le radical éthylèneoxy, n est nul ou désigne un entier allant de 1 à 100, R désigne un radical hydrocarboné choisi parmi les radicaux alkyle, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (A) plus particulièrement préféré est un motif dans lequel R' désigne H, n est égal à 10, et R désigne un radical stéaryl ($C_{18}$).

Parmi ces polymères anioniques à chaîne grasse, on préfère les polymères formés à partir de 20 à 60% en poids

d'acide acrylique et/ou d'acide méthacrylique, de 5 à 60% en poids de (méth)acrylates d'alkyles inférieurs, de 2 à 50% en poids d'éther d'allyl à chaîne grasse de formule (A), et de 0 à 1% en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC 80 et SALCARE SC90 qui sont des émulsions aqueuses à 30% d'un terpolymère réticulé d'acide méthacrylique, d'acrylate d'éthyle et de steareth-10-allyl éther (40/50/10).

-(II) les polymères comportant au moins un motif hydrophile de type acide carboxylique insaturé oléfinique, et au moins un motif hydrophobe de type ester d'alkyl ($C_{10}$-$C_{30}$) d'acide carboxylique insaturé.

De préférence, ces polymères sont choisis parmi ceux dont le motif hydrophile de type acide carboxylique insaturé oléfinique correspond au monomère de formule (B) suivante :

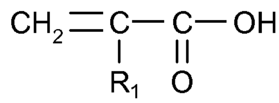

(B)

dans laquelle, $R_1$ désigne H ou $CH_3$ ou $C_2H_5$, c'est-à-dire des motifs acide acrylique, acide méthacrylique ou acide éthacrylique, et dont le motif hydrophobe de type ester d'alkyl ($C_{10}$-$C_{30}$) d'acide carboxylique insaturé correspond au monomère de formule (C) suivante :

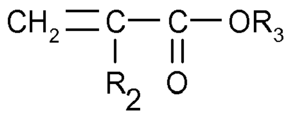

(C)

dans laquelle, $R_2$ désigne H ou $CH_3$ ou $C_2H_5$ (c'est-à-dire des motifs acrylates, méthacrylates ou éthacrylates) et de préférence H (motifs acrylates) ou $CH_3$ (motifs méthacrylates), $R_3$ désignant un radical alkyle en $C_{10}$-$C_{30}$, et de préférence en $C_{12}$-$C_{22}$.

Les esters d'alkyles ($C_{10}$-$C_{30}$) d'acides carboxyliques insaturés sont par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.

Parmi ce type de polymères anioniques à chaîne grasse, on utilisera plus particulièrement des polymères formés à partir d'un mélange de monomères comprenant :

(i) essentiellement de l'acide acrylique,

(ii) un ester de formule (C) décrite ci-dessus et dans laquelle $R_2$ désigne H ou $CH_3$, $R_3$ désignant un radical alkyle ayant de 12 à 22 atomes de carbone,

(iii) et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ce type de polymères anioniques à chaîne grasse, on utilisera plus particulièrement ceux constitués de 95 à 60% en poids d'acide acrylique (motif hydrophile), 4 à 40% en poids d'acrylate d'alkyles en $C_{10}$-$C_{30}$ (motif hydrophobe), et 0 à 6% en poids de monomère polymérisable réticulant, ou bien ceux constitués de 98 à 96% en poids d'acide acrylique (motif hydrophile), 1 à 4% en poids d'acrylate d'alkyles en $C_{10}$-$C_{30}$ (motif hydrophobe), et 0,1 à 0,6% en poids de monomère polymérisable réticulant tel que ceux décrits précédemment.

Parmi lesdits polymères ci-dessus, on préfère tout particulièrement selon la présente invention, les produits vendus par la société GOODRICH sous les dénominations commerciales PEMULEN TR1, PEMULEN TR2, CARBOPOL 1382, et encore plus préférentiellement le PEMULEN TR1, et le produit vendu par la société S.E.P.P.I.C. sous la dénomination COATEX SX.

- (III) les terpolymères d'anhydride maléique/$\alpha$-oléfine en $C_{30}$-$C_{38}$/ maléate d'alkyle tel que le produit (copolymère anhydride maléique/$\alpha$-oléfine en $C_{30}$-$C_{38}$/maléate d'isopropyle) vendu sous le nom PERFORMA V 1608 par

la société NEWPHASE TECHNOLOGIES.

- (IV) les terpolymères acryliques comprenant :

  (a) 20% à 70% en poids d'un acide carboxylique à insaturation α,β-monoéthylénique,
  (b) 20 à 80% en poids d'un monomère à insaturation α,β-monoéthylénique non-tensio-actif différent de (a),
  (c) 0,5 à 60% en poids d'un mono-uréthane non-ionique qui est le produit de réaction d'un tensio-actif monohydrique avec un monoisocyanate à insaturation monoéthylénique,

  tels que ceux décrits dans la demande de brevet EP-A-0173109 et plus particulièrement un terpolymère acide méthacrylique /acrylate de méthyle/diméthyl métaisopropényl benzyl isocyanate d'alcool béhényle éthoxylé (40OE) en dispersion aqueuse à 25%.

-(V) les copolymères comportant parmi leurs monomères un acide carboxylique à insaturation α,β-monoéthylénique et un ester d'acide carboxylique à insaturation α,β-monoéthylénique et d'un alcool gras ($C_8$-$C_{30}$)oxyalkyléné.

Préférentiellement ces composés comprennent également comme monomère un ester d'acide carboxylique à insaturation α,β-monoéthylénique et d'alcool en $C_1$-$C_4$.
A titre d'exemple de ce type de composé on peut citer l'ACULYN 22 vendu par la société ROHM et HAAS, qui est un terpolymère acide méthacrylique/acrylate d'éthyle/méthacrylate de stéaryle oxyalkyléné.
Les polymères amphiphiles non ioniques à chaîne grasse (hydrophobe) sont choisis de préférence parmi :

- (1) les celluloses modifiées par des groupements comportant au moins une chaîne grasse, comme notamment ;

  - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en $C_8$-$C_{22}$, comme le produit NATROSOL PLUS GRADE 330 CS (alkyles en $C_{16}$) vendu par la société AQUALON, ou le produit BERMOCOLL EHM 100 vendu par la société BEROL NOBEL,
  - celles modifiées par des groupes polyalkylène glycol éther d'alkyl phénol, tel que le produit AMERCELL POLYMER HM-1500 (polyéthylène glycol (15) éther de nonyl phénol) vendu par la société AMERCHOL.

- (2) les hydroxypropylguars modifiés par des groupements comportant au moins une chaîne grasse tel que le produit ESAFLOR HM 22 (chaîne alkyle en $C_{22}$) vendu par la société LAMBERTI, les produits RE210-18 (chaîne alkyle en $C_{14}$) et RE205-1 (chaîne alkyle en $C_{20}$) vendus par la société RHONE POULENC.

- (3) les copolymères de vinyl pyrrolidone et de monomères hydrophobes à chaîne grasse, avec par exemple :

  - les produits ANTARON V216 ou GANEX V216 (copolymère vinylpyrrolidone / hexadécène) vendu par la société I.S.P.
  - les produits ANTARON V220 ou GANEX V220 (copolymère vinylpyrrolidone / eicosène) vendu par la société I.S.P.

- (4) les copolymères de méthacrylates ou d'acrylates d'alkyles en $C_1$-$C_6$ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208.

- (5) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.

- (6) les polymères à squelette aminoplaste éther possédant au moins une chaîne grasse, tels que les composés PURE THIX proposés par la société SUD-CHEMIE.

- (7) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

De préférence, les polyéthers polyuréthanes comportent au moins deux chaînes grasses hydrocarbonées,

ayant de 8 à 30 atomes de carbone, séparées par une séquence hydrophile, les chaînes hydrocarbonées pouvant être des chaînes pendantes ou des chaînes en bout de séquence hydrophile.

A titre d'exemples de polyéthers polyuréthanes non ioniques à chaîne grasse utilisables dans l'invention, on peut utiliser le Rhéolate 205 à fonction urée vendu par la société RHEOX ou encore les Rhéolates 208 , 204 ou 212, ainsi que l'Acrysol RM 184, l'Aculyn ou Acrysol 44 et l'Aculyn ou Acrysol 46 de la société ROHM & HAAS [l'ACULYN 46 est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44 est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

On peut également citer le produit ELFACOS T210 à chaîne alkyle en $C_{12}$-$C_{14}$ et le produit ELFACOS T212 à chaîne alkyle en $C_{18}$ de chez AKZO, ainsi que le produit DW 1206B de chez ROHM & HAAS à chaîne alkyle en $C_{20}$ et à liaison uréthanne, proposé à 20 % en matière sèche dans l'eau.

On peut aussi utiliser des solutions ou dispersions de ces polymères notamment dans l'eau ou en milieu hydroalcoolique. A titre d'exemple, de tels polymères on peut citer, le Rhéolate 255, le Rhéolate 278 et le Rhéolate 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Les polymères amphiphiles cationiques comportant au moins une chaîne grasse (hydrophobe) utilisés peuvent notamment être choisis parmi les dérivés de cellulose quaternisée, les polyuréthanes cationiques, les polyvinyllactames cationiques, et de préférence, parmi les dérivés de cellulose quaternisée.

A titre d'exemple de polymères de ce type, on peut citer en particulier :

- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

Les radicaux alkyle portés par les celluloses ou les hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthylcelluloses quaternisées à chaînes grasses en $C_8$-$C_{30}$, les produits QUATRISOFT LM 200, QUATRISOFT LM-X 529-18-A, QUATRISOFT LM-X 529-18B (alkyle en $C_{12}$) et QUATRISOFT LM-X 529-8 (alkyle en $C_{18}$) commercialisés par la société AMERCHOL et les produits CRODACEL QM, CRODACEL QL (alkyle en $C_{12}$) et CRODACEL QS (alkyle en C18) commercialisés par la société CRODA.

La teneur en polymères épaississants, s'ils sont présents, varie habituellement de 0,05% à 5% en poids, par rapport au poids de la composition de coloration.

Selon un mode de réalisation particulièrement avantageux, la composition tinctoriale comprend un ou plusieurs agents tensioactifs. Ces derniers peuvent être indifféremment choisis, seuls ou en mélanges, parmi des tensioactifs anioniques, amphotères, non ioniques, zwittérioniques et cationiques.

En ce qui concerne les tensioactifs anioniques, on utilise habituellement les sels, en particulier les sels de métaux alcalins tels que les sels de sodium, les sels d'ammonium, les sels d'amines, les sels d'aminoalcools ou les sels de métaux alcalino-terreux, par exemple, de magnésium, des composés suivants, seuls ou en mélange :

- les alkylsulfates, les alkyléthersulfates, les alkylamidoéthersulfates, les alkylarylpolyéthersulfates ;
- les alkylsulfonates, les alkylamidesulfonates, les alkyl-arylsulfonates ;
- les alkylsulfosuccinates, les alkyléthersulfosuccinates, les alkylamide-sulfosuccinates ;
- les alkylsulfoacétates ;
- les acylsarconisates ; et les acylglutamates :

- les esters d'alkyle et d'acides polyglycoside-carboxyliques tels que les glucoside-citrates d'alkyle, les polyglycoside-tartrates d'alkyle, et les polyglycoside-sulfosuccinates d'alkyle ;
- les alkylsulfosuccinamates ;
- les acyliséthionates, les N-acyltaurates ; les acyllactylates ;
- les acides d'alkyl-D-galactoside uroniques ;
- les acides alkyléther-carboxyliques polyoxyalkylénés, les acides alkylaryléther-carboxyliques polyoxyalkylénés, les

acides alkylamidoéther carboxyliques polyoxyalkylénés ;

le groupe alkyle ou acyle (RCO-) de ces composés comportant de 10 à 24 atomes de carbone et le groupe aryle désignant de préférence un groupe phényle ou benzyle ; le nombre de groupements oxyalkylénés, et de préférence oxyéthylénés, est compris entre 2 et 50.

Pour ce qui concerne les tensioactifs non ioniques, ces derniers peuvent être avantageusement choisis parmi les composés suivants, seuls ou en mélange :

- les alcools gras polyéthoxylés, polypropoxylés ou polyglycérolés,
- les alpha-diols polyéthoxylés, polypropoxylés ou polyglycérolés, le nombre de groupements oxyde d'éthylène ou oxyde de propylène allant de 2 à 50 ; le nombre de groupements glycérol allant de 2 à 30 ;
- les condensats d'oxyde d'éthylène et d'oxyde de propylène sur des alcools gras ;
- les amides gras polyéthoxylés ayant de 2 à 30 moles d'oxyde d'éthylène ;
- les amides gras polyglycérolés comportant de 1 à 5 groupements glycérol ;
- les amines grasses polyéthoxylées ayant de 2 à 30 moles d'oxyde d'éthylène ;
- les esters éthoxylés d'acides gras du sorbitane ayant de 2 à 30 moles d'oxyde d'éthylène, les esters d'acides gras du saccharose ;
- les alkylpolyglucosides, les dérivés de N-alkylglucamine ;

ces composés comprenant au moins une chaîne alkyle ou alcényle comprenant 10 à 24 atomes de carbone.

- les copolymères d'oxyde d'éthylène et d'oxyde de propylène.

Les tensioactifs cationiques entrant dans la composition selon l'invention, peuvent notamment être choisis parmi les composés suivants, seuls ou en mélange :

- les amines grasses primaires, secondaires ou tertiaires éventuellement polyéthoxylées (2 à 30 moles d'oxyde d'éthylène) et leurs sels
- les sels d'ammonium quaternaire tels que les chlorures ou les bromures de tétraalkylammonium, d'alkylamidoalkyltrialkylammonium, de trialkylbenzyl-ammonium, de trialkylhydroxyalkylammonium, d'alkylpyridinium,
- les dérivés d'alkyl-imidazoline ;

ces composés comprenant au moins une chaîne alkyle comprenant 10 à 24 atomes de carbone.

Enfin, les tensioactifs amphotères peuvent être choisis parmi les composés suivants, seuls ou en mélange :

- les dérivés d'amines aliphatiques secondaires ou tertiaires, dans lesquels le groupe aliphatique est une chaîne linéaire ou ramifiée comportant 10 à 24 atomes de carbone et comprenant au moins un groupe anionique hydrosolubilisant tel que, par exemple, un groupe carboxylate, sulfonate, sulfate, phosphate ou phosphonate,
- les alkylbétaïnes, les sulfobétaïnes, les alkylamidoalkyl($C_6$-$C_8$)bétaïnes, les alkylamidoalkyl($C_6$-$C_8$)sulfobétaïnes ;

ces composés comprenant une au moins chaîne alkyle comprenant de 10 à 24 atomes de carbone.

De préférence, les tensioactifs sont non ioniques, anioniques ou amphotères et de manière encore plus préférée, non ioniques.

Habituellement, les agents tensioactifs représentent une quantité comprise entre 0,01 et 50% en poids, de préférence entre 0,1 et 25% en poids par rapport au poids de la composition.

La composition tinctoriale conforme à l'invention peut également renfermer divers adjuvants utilisés classiquement dans les compositions pour la teinture des cheveux, tels que par exemple des polymères anioniques, non ioniques, amphotères, zwitterioniques différents des épaississants mentionnés auparavant, ou leurs mélanges ; des agents épaississants minéraux comme notamment les argiles ; des agents antioxydants tels que par exemple l'acide ascorbique, l'acide érythorbique ; des agents réducteurs avec entre auters le sulfite, bisulfite ou métabisulfite d'ammonium, le thiolactate d'ammonium ; des agents de pénétration, des agents séquestrants comme l'éthylènediamine tétraacétique ou ses sels ; des parfums ; des agents matifiants avec par exemple les oxydes de titane ; des tampons ; des agents dispersants ; des agents filmogènes ; des céramides et des agents conservateurs.

Les adjuvants ci dessus sont en général présents en quantité comprise pour chacun d'eux entre 0,01 et 20% en poids par rapport au poids de la composition.

Le milieu cosmétiquement acceptable de la composition, qui est un milieu approprié pour la coloration des fibres kératiniques humaines, comprend de préférence de l'eau et un ou plusieurs solvants dont un ou plusieurs composés de formule (I) détaillée précédemment.

Le milieu peut éventuellement comprendre un ou plusieurs solvants organiques additionnels, différents des composés de formule(I).

A titre d'exemples de tels solvants additionnels, on peut par exemple citer, les alcanols, linéaires ou ramifiés, en $C_2$-$C_4$, tels que l'éthanol et l'isopropanol ; les polyols et éthers de polyols comme le 2-butoxyéthanol, l'hexylène glycol, le propylèneglycol, le dipropylèneglycol, le monométhyléther de propylèneglycol, le monoéthyléther et le monométhyléther du diéthylèneglycol, le glycérol ainsi que les alcools aromatiques comme l'alcool benzylique, le benzyloxyéthanol ou le phénoxyéthanol, et leurs mélanges.

Le ou les solvants additionnels peuvent être présents dans des proportions allant de préférence de 1 à 40% en poids par rapport au poids total de la composition tinctoriale, et encore plus préférentiellement de 5 à 30 % en poids.

Il est à noter que la quantité d'eau dans la composition selon l'invention est de préférence supérieure à 10% en poids par rapport au poids de la composition, et plus avantageusement supérieure ou égale à 25% en poids. De préférence, la teneur en eau est comprise entre 25 et 98% en poids par rapport au poids de la composition.

Le pH de la composition selon l'invention est compris entre 2 et 6.

Il peut être ajusté à la valeur désirée au moyen d'un ou plusieurs agents acidifiants ou d'un ou plusieurs agents alcalinisants habituellement utilisés dans le domaine.

Parmi les agents acidifiants, on peut citer à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, les carbonates alcalins, les alcanolamines telles que les mono-, di- et triéthanolamines ainsi que leurs dérivés, les hydroxydes de sodium ou de potassium et les composés de formule suivante :

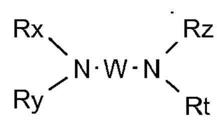

dans laquelle W est un reste propylène éventuellement substitué par un groupement hydroxyle ou un radical alkyle en $C_1$-$C_6$ ; Rx, Ry, Rz et Rt, identiques ou différents, représentent un atome d'hydrogène, un radical alkyle en $C_1$-$C_6$ ou hydroxyalkyle en $C_1$-$C_6$.

La composition selon l'invention peut comprendre également un ou plusieurs agents oxydants. On parle dans ce cas de composition prête à l'emploi.

En particulier, la composition prête à l'emploi est obtenue par mélange extemporané avant l'application, d'une composition précédemment décrite, avec au moins une composition comprenant un ou plusieurs agents oxydants.

L'agent oxydant est choisi de préférence parmi le peroxyde d'hydrogène, le peroxyde d'urée, les bromates ou ferricyanures de métaux alcalins, les sels peroxygénés comme par exemple les persulfates, les perborates et les percarbonates de métaux alcalins ou alcalino-terreux, comme le sodium, le potassium, le magnésium.

L'utilisation du peroxyde d'hydrogène est particulièrement préférée.

Cet agent oxydant est avantageusement constitué du peroxyde d'hydrogène en solution aqueuse (eau oxygénée) dont le titre peut varier, plus particulièrement, de 1 à 40 volumes, et encore plus préférentiellement de 5 à 40 volumes.

Les compostions selon l'invention peuvent résulter du mélange extemporané de plusieurs compositions.

Un autre objet de l'invention est donc constitué par un procédé de coloration de fibres kératiniques qui consiste à appliquer la composition décrite précédemment.

Conformément à un premier mode de réalisation, la composition appliquée ne comprend pas d'agent oxydant. Ce mode de réalisation est approprié dans le cas où la composition ne comprend pas de précurseur de colorant d'oxydation (bases, coupleur).

Conformément à un deuxième mode de réalisation, la composition est appliquée en présence d'au moins un agent oxydant.

Ce mode de réalisation peut être mis en oeuvre si la composition ne comprend à titre de colorants, que des colorants directs (colorant(s) à caractère hydrophobe, et éventuellement un ou des colorants directs additionnels de synthèse et/ou naturels) ou bien encore si la composition comprend un ou plusieurs colorants à caractère hydrophobe, éventuellement un ou des colorants directs additionnels de synthèse et/ou naturels, combinés à un ou plusieurs précurseurs de colorants d'oxydation (base/coupleur).

Selon une première variante de ce mode de réalisation, on applique sur les fibres la composition prête à l'emploi qui vient d'être détaillée et qui est obtenue par mélange extemporané avant l'application, d'une composition selon l'invention dépourvue d'agent oxydant avec une composition oxydante.

Selon une deuxième variante de ce mode de réalisation, on applique la composition selon l'invention dépourvue d'agent oxydant, et une composition oxydante, successivement et sans rinçage intermédiaire.
La composition oxydante mise en oeuvre comprend un ou plusieurs agents oxydants tels que définis plus haut.
Concernant les solvants organiques éventuellement présents dans la composition oxydante, on pourra se reporter à la liste indiquée auparavant dans le cadre du descriptif de la composition selon l'invention. A noter que ces solvants organiques peuvent également être choisis parmi les composés de formule (I).
Habituellement, le pH de la composition oxydante est inférieur à 7.
La composition oxydante peut se présenter sous la forme d'une solution, une émulsion ou un gel.
Elle peut éventuellement comprendre un ou plusieurs additifs utilisés classiquement dans le domaine de la coloration des fibres kératiniques humaines, en fonction de la forme galénique souhaitée. On pourra là encore se reporter à la liste des additifs donnée plus haut.
Quelle que soit le mode de réalisation retenu (avec ou sans oxydant), le mélange appliqué sur les fibres est laissé en place pour une durée, en général, de l'ordre de 1 minute à 1 heure, de préférence de 10 minutes à 30 minutes.
La température durant le procédé est classiquement comprise entre 10 et 200°C et plus particulièrement comprise entre la température ambiante (entre 15 à 25°C) et 80°C, de préférence entre la température ambiante et 60°C.
A l'issue du traitement, les fibres kératiniques humaines sont éventuellement rincées à l'eau, lavées au shampooing, rincées à nouveau à l'eau puis séchées ou laissées à sécher.
Des exemples concrets mais non limitatifs de l'invention vont maintenant être présentés.

## Exemple 1

On a préparé la composition de coloration suivante (teneurs exprimées en grammes de matière active) :

| | |
|---|---|
| HC Yellow 7 | 0,3 |
| Disperse Red 13 | 0,3 |
| Propylène carbonate | 25 |
| Ethanol | 6 |
| Hydroxyéthyl cellulose (PM 1.300.000) | 1,5 |
| Acide citrique | qsp pH 3 |
| Eau déminéralisée | qsp 100 |

Cette composition a été appliquée d'une part sur des mèches de cheveux gris naturels à 90% de blancs, d'autre part sur des mèches de cheveux gris permanentés à 90% de blancs, pendant 30 minutes à température ambiante.
A l'issue du temps de pause, les mèches ont été rincées, shampooinées puis rincées et séchées. Elles ont été teintes dans une nuance cuivrée puissante et peu sélective.

## Exemple 2

On a préparé la composition de coloration suivante (teneurs exprimées en grammes de matière active) :

| | |
|---|---|
| Solvent Black 3 | 0,25 |
| Disperse Red 17 | 0,2 |
| Solvent Orange 7 | 0,1 |
| Propylène carbonate | 25 |
| Ethanol | 6 |
| Hydroxyéthyl cellulose (PM 1.300.000) | 1,5 |
| Acide citrique | qsp pH 3 |
| Eau déminéralisée | qsp 100 |

Cette composition a été appliquée d'une part sur des mèches de cheveux gris naturels à 90% de blancs,

d'autre part sur des mèches de cheveux gris permanentés à 90% de blancs, pendant 30 minutes à température ambiante.
A l'issue du temps de pause, les mèches ont été rincées, shampooinées puis rincées et séchées. Elles ont été teintes dans une nuance marron peu sélective.

**Exemple 3**

On prépare les compositions suivantes (sauf indication contraire, les proportions indiquées en pourcentage de matière active) :

| | **Composition 1 comparative** | **Composition 2 invention** |
|---|---|---|
| Ethanol | 15g% | 15g% |
| Alcool Benzylique | 98 mmoles% | - |
| Carbonate de propylène | - | 98 mmoles% |
| Disperse Red 13 | 0,2% | 0,2% |
| Eau | qsp 100 | qsp 100 |

Au moment de l'emploi, chacune des compositions est appliquée sur des mèches de cheveux 90%blancs naturels et permanentés, à raison de 5 g de composition colorante par gramme de mèche.
Le temps de pause est de 40 minutes à 40°C.
Les mèches sont ensuite rincées à l'eau, puis lavées par le shampooing Elseve Multivitaminé 2 en 1 et séchées

Evaluation de la coloration :

La coloration est mesurée à l'aide du spectrocolorimètre MINOLTA CM2600d, en utilisant le système CIE Lab.
On calcule ensuite la sélectivité, qui met en évidence l'homogénéité de la coloration obtenue, en utilisant l'équation suivante :

$$DE^*=\sqrt{}\,(L^{*2} + a^{*2} + b^{*2})$$

$L^{*2}$, $a^{*2}$ et $b^{*2}$ représentant respectivement la différence entre les coefficients L*, a* et b* des mèches naturelles et des mèches permanentées.
Plus la valeur de DE* est faible, meilleure est la sélectivité de la coloration.

Résultats :

| | DE* |
|---|---|
| Composition 1 comparative | 14,11 |
| Composition 2 selon l'invention | 9,32 |

On observe que la composition selon l'invention permet d'obtenir une coloration significativement plus homogène que celle résultant de l'emploi de la composition comparative.

**Revendications**

1. Composition tinctoriale comprenant dans un milieu cosmétiquement acceptable, au moins un ou plusieurs colorants directs hydrophobes de logP supérieur ou égal à 2 et un ou plusieurs composés choisis parmi ceux de formule (I) suivante :

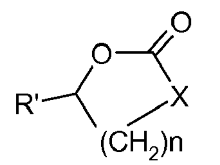

formule dans laquelle X représente un atome d'oxygène ou un groupement $CH_2$, R' un atome d'hydrogène, un radical alkyle linéaire ou ramifié en $C_1$-$C_8$, un radical hydroxyalkyle linéaire ou ramifié en $C_1$-$C_4$ ; n vaut 1, 2 ou 3 ; le pH de la composition étant compris entre 2 et 6.

**2.** Composition selon la revendication précédente, **caractérisée en ce que** le ou les colorants directs hydrophobes sont choisis parmi :

| Colorant | Structure chimique | logP |
|---|---|---|
| Disperse Red 17 | 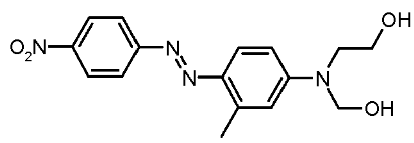 | 3.69 |
| Disperse Violet 1 | 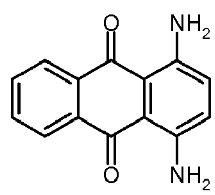 | 3.0 |
| HC Yellow 7 | 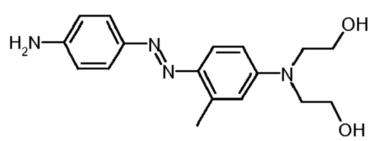 | 2.38 |
| Disperse Blue 377 | 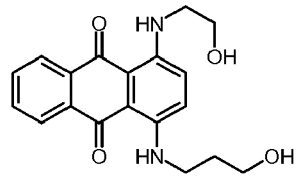 | 3.21 |
| Disperse Red 13 | 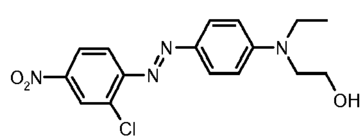 | 5.22 |
| Disperse Green 9 | 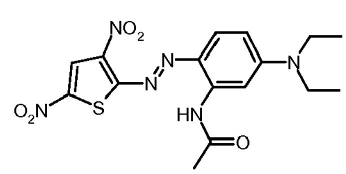 | 4.23 |
| Solvent Black 3 | 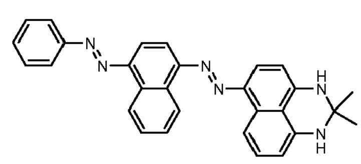 | 7.50 |

(suite)

| Colorant | Structure chimique | logP |
|---|---|---|
| Disperse Blue 148 | 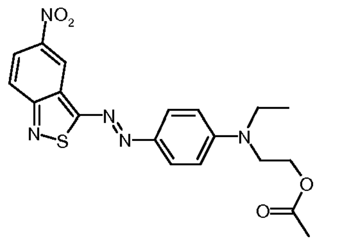 | 4.81 |
| Disperse Violet 63 | 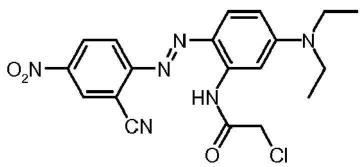 | 5.30 |
| Disperse Blue 60 | 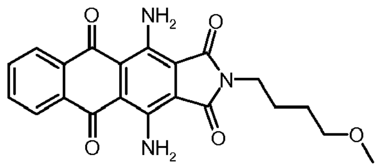 | 3.38 |
| Disperse Blue 14 | 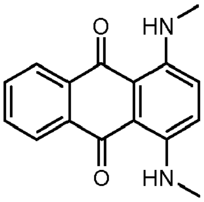 | 4.25 |
| Solvent Orange 15 | 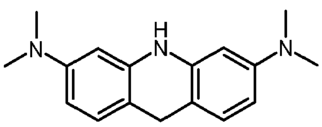 | 3.90 |
| Solvent Orange 7 | 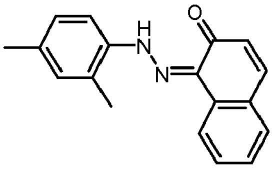 | 4.40 |
| Solvent Blue 14 | 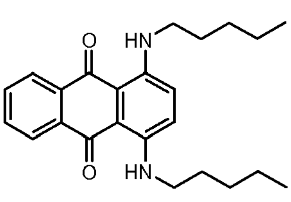 | 8.18 |
| Disperse Yellow 82 | 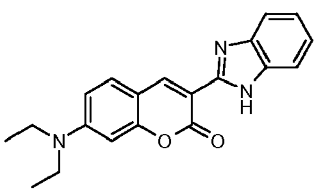 | 3.68 |

3. Composition selon l'une quelconque des revendications précédentes, **caractérisée en ce que** la teneur en colorant (s) direct(s) hydrophobe(s) varie de 0,01 à 10 % en poids par rapport au poids de la composition.

4. Composition selon l'une quelconque des revendications précédentes, **caractérisée en ce que** le composé de formule (I) est choisi parmi les carbonates d'alkylène de formule suivante :

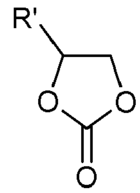

(Ia)

dans laquelle R' représente un atome d'hydrogène, un radical alkyle linéaire ou ramifié en $C_1$-$C_8$, de préférence en $C_1$-$C_4$, un radical hydroxyalkyle linéaire ou ramifié en $C_1$-$C_4$, de préférence en $C_1$-$C_2$.

5. Composition selon l'une quelconque des revendications 1 à 3, **caractérisée en ce que** le composé de formule (I) est choisi parmi les lactones de formule suivante :

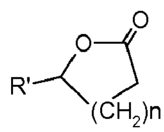

(Ib)

dans laquelle R' représente un atome d'hydrogène, un radical alkyle linéaire ou ramifié en $C_1$-$C_8$, un radical hydroxyalkyle linéaire ou ramifié en $C_1$-$C_4$, n vaut 1, 2 ou 3.

6. Composition selon l'une quelconque des revendications précédentes, **caractérisée en ce que** la teneur en composé de formule (I) représente de 0,5 à 50 % en poids par rapport au poids de la composition.

7. Composition selon l'une quelconque des revendications précédentes, **caractérisée en ce que** la composition comprend au moins un colorant naturel.

8. Composition selon la revendication précédente, **caractérisée en ce que** le ou les colorants naturels sont choisis parmi la lawsone, la juglone, l'alizarine, la purpurine, l'acide carminique, l'acide kermésique, la purpurogalline, la protocatéchaldéhyde, l'indigo, l'isatine, la curcumine, la spinusoline, les chlorophylles, les chlorophyllines, les orcéines, l'hématéine, l'hématoxyline, la braziline, la braziléine, les colorants du carthame, les flavonoïdes, les anthocyanes, les caroténoïdes, les tanins, le sorgho et le carmin de cochenille.

9. Composition selon l'une quelconque des revendications 7 ou 8, **caractérisée en ce que** la teneur en colorant(s) naturel(s) représente de 0,01 à 10 % en poids par rapport au poids de la composition.

10. Composition selon l'une quelconque des revendications précédentes, **caractérisée en ce qu'**elle comprend au moins un colorant supplémentaire, choisi parmi les colorants directs différents des colorants à caractère hydrophobe, les précurseurs de colorant d'oxydation, ou leurs combinaisons.

11. Composition selon l'une quelconque des revendications précédentes, **caractérisée en ce qu'**elle comprend de l'eau et éventuellement un ou plusieurs solvants organiques additionnels différents des composés de formule (I).

12. Composition selon l'une quelconque des revendications précédentes, **caractérisée en ce qu'**elle comprend un ou plusieurs agents oxydants.

13. Procédé de coloration consistant à appliquer sur les fibres kératiniques humaines, une composition selon l'une quelconque des revendications précédentes.

Europäisches Patentamt
European Patent Office
Office européen des brevets

## RAPPORT DE RECHERCHE EUROPEENNE

Numéro de la demande
EP 09 15 3980

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 3 565 571 A (REESE GUNTER ET AL) 23 février 1971 (1971-02-23)<br>* colonne 1, ligne 31 - ligne 33 *<br>* colonne 1, ligne 48 - ligne 68 *<br>* colonne 2, ligne 23 - ligne 39 *<br>* colonne 2, ligne 45 - ligne 50 *<br>* exemple 6 *<br>----- | 1-13 | INV.<br>A61K8/49<br>A61K8/58<br>A61Q5/06<br>A61K8/41<br>A61K8/40 |
| X | US 2002/144356 A1 (KAWAI TETSUYA [JP] ET AL) 10 octobre 2002 (2002-10-10)<br>* alinéas [0008] - [0012], [0017], [0023], [0029] *<br>----- | 1-13 | |
| A | DWECK A.C.: "Natural ingredients for colouring and styling"<br>INTERNATIONAL JOURNAL OF COSMETIC SCIENCE, vol. 24, 2002, pages 287-302, XP002500910<br>* le document en entier *<br>----- | 1-13 | |

DOMAINES TECHNIQUES RECHERCHES (IPC)

A61Q
A61K

Le présent rapport a été établi pour toutes les revendications

| Lieu de la recherche | Date d'achèvement de la recherche | Examinateur |
|---|---|---|
| Munich | 6 juillet 2009 | Simon, Frédéric |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..........
& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 09 15 3980

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

06-07-2009

| Document brevet cité au rapport de recherche | Date de publication | Membre(s) de la famille de brevet(s) | Date de publication |
|---|---|---|---|
| US 3565571 A | 23-02-1971 | DE 1492194 A1 | 04-12-1969 |
| US 2002144356 A1 | 10-10-2002 | DE 10200185 A1 | 11-07-2002 |
| | | JP 4181749 B2 | 19-11-2008 |
| | | JP 2002205927 A | 23-07-2002 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9515144 A
- WO 9501772 A
- EP 714954 A
- FR 2189006
- FR 2285851
- FR 2140205
- EP 1378544 A
- EP 1674073 A
- GB 1026978 A
- GB 1153196 A
- FR 2801308
- DE 2359399
- JP 63169571 A
- JP 5063124 A
- EP 0770375 A
- WO 9615765 A
- DE 3843892
- DE 4133957
- WO 9408969 A
- WO 9408970 A
- FR 2733749 A
- DE 19543988
- FR 2886136
- EP 0173109 A


### Littérature non-brevet citée dans la description


- **Meylan ; Howard.** Atom / Fragment contribution method for estimating octano/-water partition coefficient. *J. Pharm. Sci.,* 1995, vol. 84, 83-92
- **Walter NOLL.** Chemistry and Technology of Silicones. Academic Press, 1968
- **G. Fonnum ; J. Bakke ; Fk. Hansen.** *Colloid Polym. Sci,* 1993, vol. 271, 380.389